# EUROPEAN PATENT APPLICATION

(11) **EP 4 534 034 A1**
(43) Date of publication of application: **09.04.2025**
(21) Application number: 22943365.1
(22) Date of filing: 13.07.2022
(51) Int. Cl.: A61B 34/30, A61B 90/40

(54) **SURGICAL ROBOT DEVICE AND OPERATING METHOD THEREFOR**

(30) Priority: 26.05.2022 US 202217825630; 07.07.2022 CN 202210803401
(71) Applicant: Shenzhen Tonglu Technology Co., Ltd, Shenzhen, Guangdong 518000 (CN)
(72) Inventor: MEGLAN, Dwight Alan, Lincoln, Massachusetts 01773 (US); CAO, Yunfei, Shenzhen, Guangdong 518000 (CN); XIN, Ann, Lincoln, Massachusetts 01773 (US)
(74) Representative: Fish & Richardson P.C.
(86) International application number: PCT/CN2022/105332
(87) International publication number: WO 2023/226174

(57) **Abstract**

A surgical robot device (100), comprising a delivery module (10) capable of moving towards or away from a subject. The delivery module (10) comprises a driving assembly (16) and an operating assembly (14), wherein the operating assembly (14) and the driving assembly (16) are respectively located on a sterile side and a non-sterile side that are isolated from each other; and the operating assembly (14) moves and/or rotates under contactless remote control of the driving assembly (16) to drive an endovascular medical instrument (32) to be delivered into or withdrawn from the body of the subject. Contactless power transmission is achieved by means of remote control. In addition, the driving assembly (16) is arranged on the non-sterile side, thereby achieving sterile isolation and reuse thereof.

## Description

### Technical Field

The present disclosure relates to a medical surgical robot apparatus, particularly to a surgical robot apparatus for operating endo-tools such as catheters and guidewires on a clinical subject and a method of operating the same.

### Background Art

In existing practice, during an operation involving the use of a catheter, the operator must stand near a clinical subject to manipulate, for example, an endo-tool, such as a guidewire, guide catheter, or angioplasty catheter, into or out of the clinical subject including the vascular system. Due to many clinical procedures involving operators spending extended amounts of time standing by the clinical subject while using a fluoroscope, long term exposure to radiation is not desirable. Multiple shifts or limited hours are often applied in order to stay within the exposure requirements which increase the operational cost yet still subject operators to the undesirable conditions.

In addition, not separating between the sterile parts coming into contact with sterile patient and the non-sterile portion of the surgical devices proves to be extremely costly because the non-sterile portions are often expensive mechanical driving gears that cannot be re-sterilized and must be discarded after a single use. Even if re-sterilization of the mechanical driving gears is possible, it is often prohibitively expensive.

Furthermore, robotic or electromechanical devices in existing practice performing catheter operations are bulky and expensive.

### Contents of Invention

Accordingly, to overcome deficiencies in the prior art, it is necessary to provide a new surgical robot apparatus performing sterile isolation and a method of operation thereof.

The present disclosure provides a surgical robot apparatus, which comprises a robot moving hand configured to move towards or away from a clinical subject. The robot moving hand comprises a moving hand actuator and a moving hand manipulator, the moving hand actuator and the moving hand manipulator are respectively located on a non-sterile side and a sterile side, which are isolated from each other, the moving hand manipulator is spatially contactlessly controlled by the moving hand actuator to move and/or rotate, thereby the moving hand manipulator is configured to maneuver an endo-tool to be inserted into or withdrawn from a clinical subject.

Preferably, the moving hand manipulator is spatially contactlessly controlled by the moving hand actuator to move and/or rotate by means of magnetic driving.

Preferably, the moving hand manipulator is spatially contactlessly controlled by the moving hand actuator to move and/or rotate by means of electromagnetic induction driving.

Preferably, the moving hand manipulator is spatially contactlessly controlled by the moving hand actuator to move and/or rotate by means of electric field coupling driving.

Preferably, the moving hand manipulator is spatially contactlessly controlled by the moving hand actuator to move and/or rotate by means of direct current resonance driving.

Preferably, the moving hand actuator comprises at least one magnetic driving gear, the moving hand manipulator comprises at least one magnetic induction gear corresponding to the magnetic driving gear, the magnetic induction gear is spatially contactlessly controlled by the magnetic driving gear.

Preferably, the moving hand manipulator comprises a permanent magnetic induction rotating gear and a permanent magnetic induction holding gear, the moving hand actuator comprises a permanent magnetic driving gear and the first electric magnet, the permanent magnetic induction holding gear is spatially contactlessly controlled to not move by the first electric magnet, the permanent magnetic induction rotating gear is spatially contactlessly controlled to rotate by the permanent magnetic driving gear.

Preferably, the surgical robot apparatus further comprises collet chuck nestled between the permanent magnetic induction rotating gear and the permanent magnetic induction holding gear, the collet chuck is configured for an endo-tool threaded therethrough, when the permanent magnetic induction rotating gear and the permanent magnetic induction holding gear rotate relative to each other, the collet chuck is in a closed position to hold an endo-tool or the collet chuck is in an open position to release an endo-tool.

Preferably, the collet chuck comprises a collet barrel and a collet spindle for coaxially positioned inside the collet barrel, the collet barrel has inner threads on one end and a collet nozzle on the other end, the collet spindle has outer threads coupling with the inner threads and a collet tweezer corresponding to the elastic collet nozzle, when the collet spindle is threaded more to the other end of the collet barrel and positioned inside the collet barrel, the collet tweezer is pushed more into the collet nozzle, forcing it elastically close upon endo-tool and hold onto an endo-tool, or when the collet spindle is threaded away from the other end of the collet barrel and withdraws from the collet barrel, the collet tweezer is pulled out of the collet nozzle, the collet tweezer is elastically restored and releases an endo-tool.

Preferably, the collet barrel protrudes on one of the permanent magnetic induction rotating gear and the permanent magnetic induction holding gear, the collet spindle is formed on the other of the permanent magnetic induction rotating gear and the permanent magnetic induction holding gear.

Preferably, the collet tweezer comprises a plurality of pieces.

Preferably, the moving hand manipulator comprises a permanent magnetic induction rotating gear, a permanent magnetic induction holding gear and a collet chuck, when the permanent magnetic induction holding gear is stationary to not move and the permanent magnetic induction rotating gear rotates relative to the permanent magnetic induction holding gear, the collet chuck is in a closed position to hold an endo-tool and in an open position to release an endo-tool.

Preferably, when the collet chuck is in a closed position to hold an endo-tool and the permanent magnetic induction holding gear is released, the permanent magnetic induction rotating gear and the permanent magnetic induction holding gear synchronously rotate together with an endo-tool.

Preferably, the surgical robot apparatus further comprises a track, when the moving hand actuator travels along the track, the moving hand manipulator is moved by spatially contactless control of the moving hand actuator.

Preferably, the moving hand actuator and the moving hand manipulator are each provided with magnetic components, magnetic attraction of the magnetic components is configured to engage the moving hand manipulator with the moving hand actuator together.

Preferably, when the collet chuck is in a closed position to hold an endo-tool and the permanent magnetic induction holding gear is released, the moving hand actuator travels along the track, the moving hand manipulator synchronously move together with the moving hand actuator by magnetic attraction of the magnetic components of the moving hand actuator and the moving hand manipulator, thereby robot moving hand pushing an endo-tool towards or withdrawing an endo-tool from a clinical subject along the track.

Preferably, the moving hand manipulator further comprises two cylindrical bearings respectively projecting on the permanent magnetic induction rotating gear and the permanent magnetic induction holding gear, the two cylindrical bearings are respectively supported by two bearing hangers, the collet chuck is supported by another bearing hanger between the two bearing hangers.

Preferably, the moving hand actuator is housed in a base housing, which is on the non-sterile side, the moving hand manipulator is housed in manipulator housing, which is on the sterile side, the manipulator housing is detachably mounted on the base housing and linearly move relative to the base housing.

Preferably, a sterile barrier is placed between the sterile side and the non-sterile side and configured to separate the sterile side from the non-sterile side, the base housing stays underneath the sterile barrier.

Preferably, the moving hand manipulator comprises a permanent magnetic induction rotating gear and a permanent magnetic induction holding gear, the moving hand actuator comprises the first permanent magnetic driving gear and the second permanent magnetic driving gear, the permanent magnetic induction holding gear and the permanent magnetic induction rotating gear are spatially contactlessly controlled to rotate by the first permanent magnetic driving gear and the second permanent magnetic driving gear, respectively.

Preferably, the surgical robot apparatus further comprises a robot stationary hand, the robot stationary hand comprises a support member on the sterile side for supporting an endo-tool.

Preferably, the surgical robot apparatus further comprises a robot stationary hand, the robot stationary hand comprises a stationary hand holder on the sterile side and a controller on the non-sterile side, the stationary hand holder is spatially contactlessly controlled by the controller to hold or release an endo-tool.

Preferably, the robot stationary hand is stationary to not move between the robot moving hand and a clinical subject, when the robot stationary hand releases an endo-tool, the moving hand manipulator holds and maneuvers an endo-tool to be inserted into or withdrawn from a clinical subject.

Preferably, the robot stationary hand is stationary to not move between the robot moving hand and a clinical subject, when the robot stationary hand releases an endo-tool, when the robot stationary hand holds an endo-tool, the moving hand manipulator releases an endo-tool and moves towards or away from a clinical subject.

Preferably, the stationary hand holder is spatially contactlessly controlled by the controller to hold or release by magnetic means.

Preferably, the controller is the second electric magnet.

Preferably, the stationary hand holder comprises a stationary hand cylinder, a stationary hand clamp slidably placed in the stationary hand cylinder, and a stationary hand magnetic element fixed on the stationary hand clamp, the stationary hand magnetic element is spatially contactlessly controlled by the second electric magnet to be held or released, thereby the stationary hand clamp sliding in the stationary hand cylinder close and holding endo-tool or away from endo-tool and releasing endo-tool.

Preferably, the robot moving hand is placed between the robot stationary hand and a clinical subject, when the robot stationary hand and the robot moving hand move together with an endo-tool into a clinical subject or withdraw together with an endo-tool from a clinical subject a certain distance, after the robot moving hand moves towards or away from the robot stationary hand a certain distance, the robot stationary hand and the robot moving hand move again together with an endo-tool into a clinical subject or withdraw together with an endo-tool from a clinical subject a certain distance.

Preferably, the surgical robot apparatus further comprises a robot stationary hand between the robot moving hand and a clinical subject, the robot stationary hand comprises two opposite frictional rollers, which oppositely hold an endo-tool, when the moving hand manipulator holds and maneuvers an endo-tool to be inserted into or withdrawn from a clinical subject, the endo-tool is free to move relative to the frictional rollers.

Preferably, the surgical robot apparatus further comprises at least two position sensors, which are configured to sense the presence of the robot moving hand reaching the distal maximum position or the proximal maximum position.

The surgical robot apparatus provided by the present disclosure not only realizes contactless transmission through spatial control, but also allows the moving hand actuator placed on the non-sterile side, thereby performing sterile isolation and reusable.

The present disclosure further provides a method of operating a surgical robot apparatus, which comprises a robot moving hand having a moving hand actuator and a moving hand manipulator, the operating method comprising:
allowing the moving hand manipulator placed on the sterile side and the moving hand actuator placed on the non-sterile side apart from the sterile side; and
allowing the moving hand manipulator moving and/or rotating by spatially contactless controlling of the moving hand actuator, and thereby the moving hand manipulator together with an endo-tool being inserted into a clinical subject or withdrawn from a clinical subject.

Preferably, the moving hand manipulator is spatially contactlessly controlled by the moving hand actuator to move and/or rotate by means of magnetic driving.

Preferably, the moving hand manipulator is spatially contactlessly controlled by the moving hand actuator to move and/or rotate by means of electromagnetic induction driving.

Preferably, the moving hand manipulator is spatially contactlessly controlled by the moving hand actuator to move and/or rotate by means of electric field coupling driving.

Preferably, the moving hand manipulator is spatially contactlessly controlled by the moving hand actuator to move and/or rotate by means of direct current resonance driving.

Preferably, the moving hand actuator comprises at least one magnetic driving gear, the moving hand manipulator comprises at least one magnetic induction gear corresponding to the magnetic driving gear, the magnetic induction gear is spatially contactlessly controlled by the magnetic driving gear.

Preferably, the surgical robot apparatus further comprises a track, when the moving hand actuator travels along the track, the moving hand manipulator is moved by spatially contactless control of the moving hand actuator.

Preferably, the moving hand actuator and the moving hand manipulator are each provided with magnetic components, magnetic attraction of the magnetic components is configured to engage the moving hand manipulator with the moving hand actuator together.

Preferably, the surgical robot apparatus further comprises a robot stationary hand, the robot stationary hand comprises a stationary hand holder on the sterile side and a controller on the non-sterile side, the stationary hand holder is spatially contactlessly controlled by the controller to hold or release an endo-tool.

Preferably, the operation method comprises: allowing the robot stationary hand still between the robot moving hand and a clinical subject, when the robot stationary hand holds an endo-tool still and the moving hand manipulator releases the endo-tool, the robot stationary hand moves towards or away from a clinical subject.

The method of operating a surgical robot apparatus provided by the present disclosure realizes spatially contactless control/drive, there avoids the entry or leakage of body fluid or contamination during controlling/driving process of directly or indirectly contact control/drive mechanism, and prevents the moving hand actuator being contaminated, and provides protection against it to the maximum extent.

### Description of Figures

Fig. 1 is a perspective view of an embodiment of a surgical robot apparatus (its housing is not shown) for operation of an endo-tool on a clinical subject in accordance with the present disclosure.
Fig. 2 is a left view of Fig. 1, with the housing of the surgical robot apparatus shown.
Fig. 3 is a right view of Fig. 1, with the housing of the surgical robot apparatus shown.
Fig. 4 is a cross-sectional view of Fig. 2 along A-A.
Fig. 5 is a front view of Fig. 1, with the housing of the surgical robot apparatus shown.
Fig. 6 is a top view of the moving hand manipulator of Fig. 5.
Fig. 6a is a cross-sectional view of Fig. 6 along B-B, with collet chuck in an open position.
Fig. 6b is a cross-sectional view of Fig. 6 along B-B, with collet chuck in a closed position.
Fig. 7 is another view of the moving hand manipulator of Fig. 1, showing the operation from open to closed of the collet chuck of the moving hand manipulator.
Fig. 8 is yet another view of the moving hand manipulator of Fig. 1, showing the rotation of the collet chuck of the moving hand manipulator.
Fig. 9 is a top view of the robot stationary hand of Fig. 5.
Fig. 9a is a cross-sectional view of Fig. 9 along C-C, with the robot stationary hand in an open position.
Fig. 9b is a cross-sectional view of Fig. 9 along C-C, with the robot stationary hand in a closed position.
Fig. 10 is another view of Fig. 4, with an endo-tool added and held by the moving hand manipulator for inserting the endo-tool into a clinical subject.
Fig. 11 is yet another view of Fig. 4, with an endo-tool added and held by the moving hand manipulator for removing an endo-tool from a clinical subject.
Fig. 12 is yet another view of Fig. 4, with the robot moving hand in an initial position of sensor triggers.
Fig. 13 is yet another view of Fig. 4, with the robot moving hand in an ultimate position of sensor triggers.
Fig. 14 is an assembly view of three of the surgical robot apparatus in Fig. 5.
Figs. 15a and 15b are schematic views of operation of an embodiment of two of the robot moving hand of a surgical robot apparatus in accordance with the present disclosure.
Fig. 16 is a schematic view of operation of another embodiment of two of the robot moving hand of a surgical robot apparatus in accordance with the present disclosure.
Fig. 17 is a schematic view of operation of yet another embodiment of two of the robot moving hand of a surgical robot apparatus in accordance with the present disclosure.
Fig. 18 is a perspective view of another embodiment of a surgical robot apparatus (its housing is not shown) for operation of an endo-tool on a clinical subject in accordance with the present disclosure.

### Specific Embodiments for Carrying out the Invention

For clear description and better understanding of the technical problem to be solved, technical solutions, and advantages of the present disclosure, the present disclosure is further described in detail in conjunction with the accompanying drawings and specific embodiments. It should be understood that the specific embodiments herein described are only used to explain the present disclosure, not to limit the present disclosure.

In the present disclosure, "proximal end" means the end near the operator, "distal end" means the end away from the operator. Unless otherwise clearly specified and limited, the terms "installation", "attachment", and "connection" should be understood in a broad sense, for example, it may be a fixed attachment, a detachable connection, or an integral body. It may also be an attachment that can move relatively. It may also be a mechanical connection or an electrical connection. It may be a direct or indirect connection through an intermediary. It may be an internal communication of two components or the interaction relationship between two components. The specific meanings of the above terms used in the present disclosure are to be understood by those skilled in the art according to specific situations.

The surgical robot apparatus of the present disclosure can be used for insertion or withdrawal of endo-tools, also refer to as intraluminal surgical devices. It should be understood that the term "intraluminal" includes lumens such as natural lumens, panvascular lumens, and organ lumens. The term "intraluminal surgical device" may refer to any shape or any kind of catheter, guide wire, guide catheter, angioplasty catheter, or endoscope, various laparoscopes, tube mirrors, etc., including those suitable for vascular intervention, any catheter, guide wire, or consumable device for electrophysiology, structural heart disease and other procedures. Therefore, the surgical robot apparatus of the present disclosure may also be referred to as a "catheter robot". It should be appreciated that the scope and spirit of the present disclosure is not limited to these examples of the present disclosure.

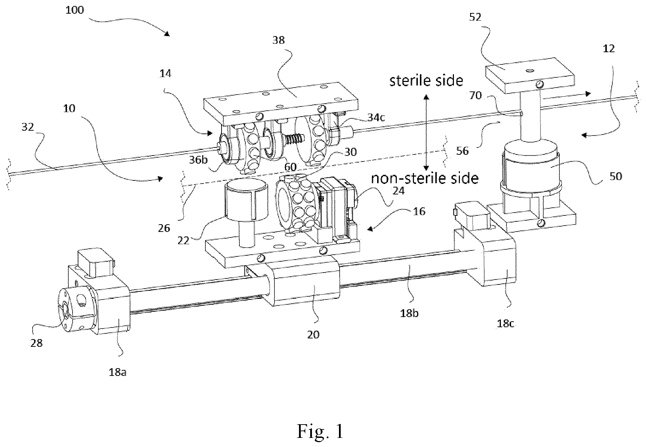

In order to facilitate the explanation of the operation of surgical robot apparatus 100, manual operations in the existing art is herein referred to as a comparison. Manually, operator usually stands to the left of the clinical subject in Fig. 1 (not shown), in place of surgical robot apparatus 100, to manipulate endo-tool 32 into or out of the clinical subject. The insertion of endo-tool 32 involves mainly two kinds of forces. One is the force of pushing/pulling the axially stiff and bending flexible endo-tool 32, the other is the torquing of the rotationally stiff endo-tool 32, often while pushing. The operation involves the coordination of two hands of the operator to transfer endo-tool 32 more into the clinical subject, or more out from the clinical subject.

More specifically, for example, assuming the operator stands with his/her back facing the reader of Fig. 1, during the insertion of endo-tool 32, the operator lets the right hand open to release endo-tool 32. At the same time, the operator uses the left hand (sometimes both hands) to push, pull or torque the rigidly flexible endo-tool 32. When one segment of endo-tool 32 is pushed into the clinical subject, resulting in the left hand being moved closer to the right hand, the operator would need to close the right hand, holding endo-tool 32 to not move, and at the same time, move release and move the left hand further down to the left to allow it to be ready to push and/or rotate the next segment of endo-tool 32 into the clinical subject. Similar coordination among the left hand and the right hand applies during the process of removing endo-tool 32 from the clinical subject. That is, during the time the left hand is pulling or torquing, the right hand is open and lets loose endo-tool 32. During the time the left hand is transferring from one segment of endo-tool 32 to another, the right hand is closed, stopping endo-tool 32 from moving while the left hand repositions to facilitate the continued withdrawal of the endo-tool 32, such as a catheter.

Fig. 1 is a perspective view of a surgical robot apparatus for operation of an endo-tool on a clinical subject in accordance with the present disclosure. For the clarity of the overall operational environment of the surgical robot apparatus, some elements not shown in the drawings, are important to the present disclosure. These include a clinical subject to the far right hand side of the diagram of Fig. 1, and a sterile barrier enveloping the whole moving hand actuator 16, shown as a line sterile barrier 26 but rather is an enveloping barrier. The sterile barrier 26 is preferably disposable.

As shown in Fig. 1, surgical robot apparatus 100 according to the present disclosure comprises a robot moving hand 10 and a robot stationary hand 12. Robot moving hand 10 is configured to move linearly on a track 18b towards or away from the clinical subject, which is not shown but situated further to the far right hand side of the diagram of Fig. 1. Robot moving hand 10, or the moving hand, for short, further comprises a moving hand actuator 16 and a moving hand manipulator 14. Moving hand actuator 16 may have one or two sub-actuators, each of which is controlled by an operator (not shown), preferably by means of remote controls. Moving hand manipulator 14 maneuvers an endo-tool 32 to be inserted to or withdrawn from the clinical subject, being spatially contactlessly controlled by moving hand actuator 16. A force linearly along endo-tool 32 and/or a torquing axial rotation on endo-tool 32 are applied to achieve the above.

Robot stationary hand 12 is linearly stationary between the clinical subject and robot moving hand 10. Robot stationary hand 12 is configured to be stationary relative to the clinical subject and track 18b. Robot stationary hand 12 is configured to either hold or release endo-tool 32 that is threaded through it. Robot stationary hand 12 comprises a stationary hand holder and a controller. The stationary hand holder is configured to either hold or release endo-tool 32 that is threaded through it. The controller is configured to spatially and contactlessly control the stationary hand holder hold or release endo-tool 32. In the embodiment, the controller can be a second electric magnet 50, the stationary hand holder can be a magnetic stationary hand holder controlled spatially and contactlessly by the second electric magnet 50. Additionally, robot stationary hand 12 is to support endo-tool 32 along linear extension path of endo-tool 32, preventing it from tortuosity. In alternative embodiment, robot stationary hand 12 includes a support member for supporting endo-tool 32.

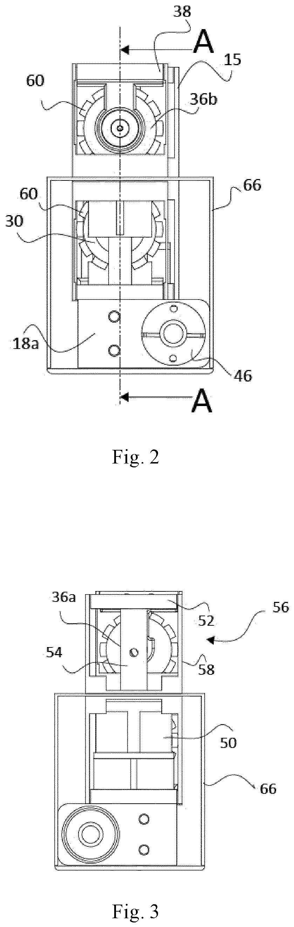

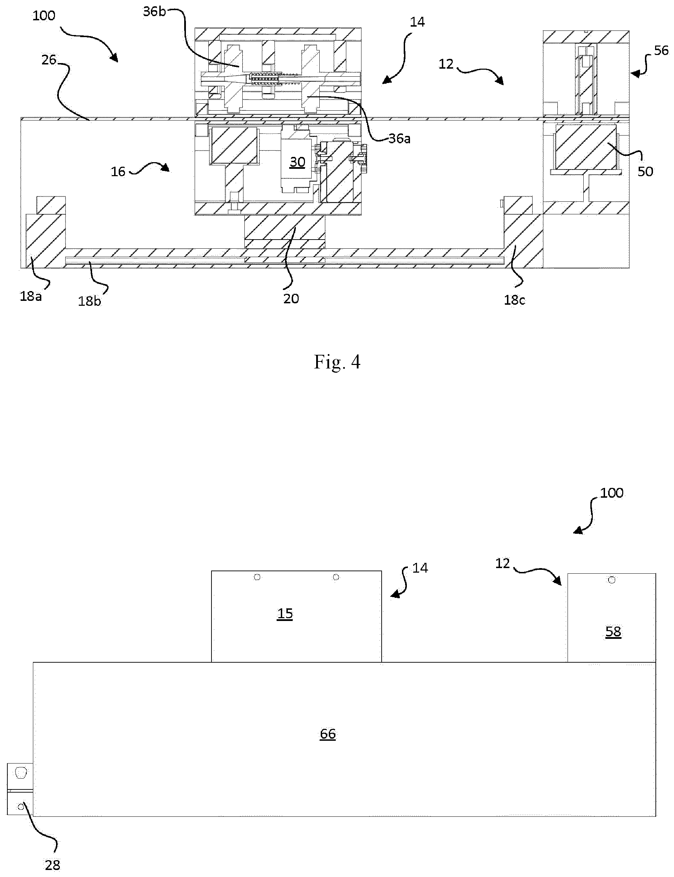

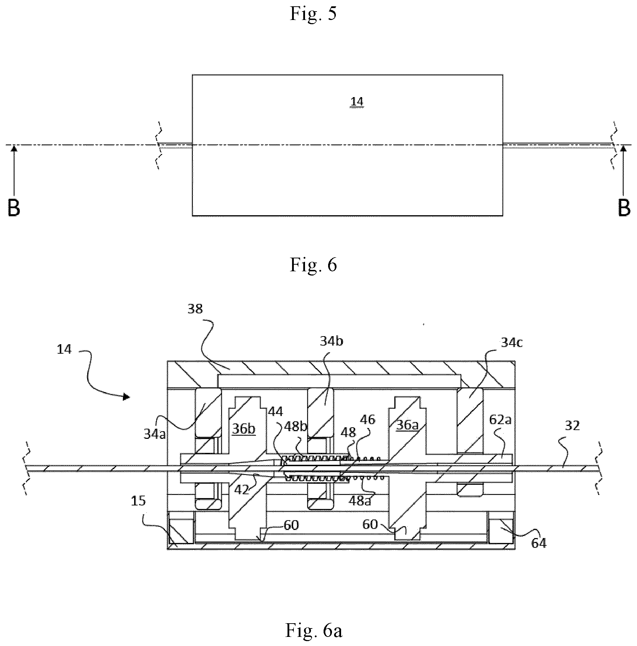

Fig. 2 is a left view of the surgical robot apparatus (with a housing added) in accordance with the present disclosure. Fig. 3 is a right view of the surgical robot apparatus (with a housing added) in accordance with the present disclosure. Fig. 4 is a cross-sectional view of the surgical robot apparatus (with a housing added) along A-A in accordance with the present disclosure. Fig. 5 is a front view of the surgical robot apparatus (with a housing added) in accordance with the present disclosure.

Referring to Figs. 1, 4 and 5, a sterile barrier 26 configured to separate a sterile side from a non-sterile side, as shown in Fig. 1, is used to wrap moving hand actuator 16 and the second electric magnet 50 in a sterilely concealed or blocking manner. This is to prevent anything crossed from non-sterile side to the sterile side or vice versa as shown in Fig. 1. Furthermore, body fluid or contamination from moving hand manipulator 14 or stationary hand clamp 70 on the sterile side is prevented from entering into the non-sterile side as well. As can be seen, moving hand actuator 16 and the second electric magnet 50 are on the non-sterile side, and moving hand manipulator 14 and stationary hand clamp 70 are on the sterile side to maneuver an endo-tool 32 contacting patients or the clinical subject. Manipulator housing 15 and stationary hand holder housing 58 come to the clinical procedure pre-sterilized and stay sterile. Base housing 66 is on the non-sterile side and stays underneath a sterile barrier 26. Manipulator housing 15 can be detachably mounted on base housing 66 and linearly move relative to base housing 66, stationary hand holder housing 58 is fixedly mounted on base housing 66. More specifically, by means of engagement mechanisms such as dovetail grooves and sliders between them, thus manipulator housing 15 may be linearly move relative to base housing 66. When actuator platform 20 carrying moving hand actuator 16 moves between backend track stand 18a and track frontend track stand 18c, connecting moving hand manipulator 14 and moving hand actuator 16 together by means of a connecting structure, such as magnetic components, allows moving hand manipulator 14 to move synchronously with moving hand actuator 16 together. A disposable sterile barrier 26 is preferably to be changed for each patient or clinical subject.

As shown in Figs. 1~5, moving hand manipulator 14 is housed in manipulator housing 15, with a moving hand hanger support 38, preferably on the top. Moving hand actuator 16 is housed in base housing 66. Track 18b is positioned in base housing 66 and supported by backend track stand 18a and frontend track stand 18c of base housing 66, and actuator platform 20 carrying moving hand actuator 16 moves between backend track stand 18a and track frontend track stand 18c. A track motor coupler 28 or a pair of track motor couplers 28 drive a transfer system such as rod and nut of screw assembly, gear linkage transfer system, rack and pinion of transfer system or synchronous belt and pulley of transfer system, thereby moving with actuator platform 20 back and forth between backend track stand 18a and frontend track stand 18c. For smooth operation of actuator platform 20, a rail and slider mechanism can be added between actuator platform 20 and base housing 66 for guiding and balancing. Preferably, manipulator housing 15 is disposable, and base housing 66 is non- disposable.

In this embodiment, connecting structure between moving hand manipulator 14 and moving hand actuator 16 may be permanent magnets. Namely, moving hand manipulator 14 and moving hand actuator 16 are each provided with permanent magnets and are fixedly positioned to each other by magnetic attraction. More specifically, permanent magnets of moving hand manipulator 14 are secured on moving hand manipulator housing 15, while permanent magnets of moving hand actuator 16 are placed closer to permanent magnets of moving hand manipulator 14. When moving hand manipulator 14 is mounted on base housing 66 through moving hand manipulator housing 15, moving hand manipulator 14 and moving hand actuator 16 are held together by means of magnetic attraction of permanent magnets of them. When moving hand actuator 16 travels, moving hand manipulator 14 and moving hand manipulator housing 15 outside base housing 66 can be magnetically driven to move in the same direction. Thus, contactless control/drive is achieved in a completely enclosed space. There is no need to design a complex structure on base housing 66 and moving hand manipulator housing 15 to install a transmission mechanism and achieve drive by means of contact transmission mechanism between moving hand manipulator 14 and moving hand actuator 16. It completely avoids the entry or leakage of body fluid or contamination during drive process of directly or indirectly contact transmission mechanism. Permanent magnets on moving hand manipulator 14 and moving hand actuator 16 can also play a role in positioning moving hand manipulator housing 15 and base housing 66 against each other to achieve a mounting effect. Of course, in alternative embodiments, connecting structure between moving hand manipulator 14 and moving hand actuator 16 may be electric magnets, or may be a linkage fixed to moving hand actuator 16, which extends out of base housing 66 and further extends to be secured to moving hand manipulator housing 15(deterioration example).

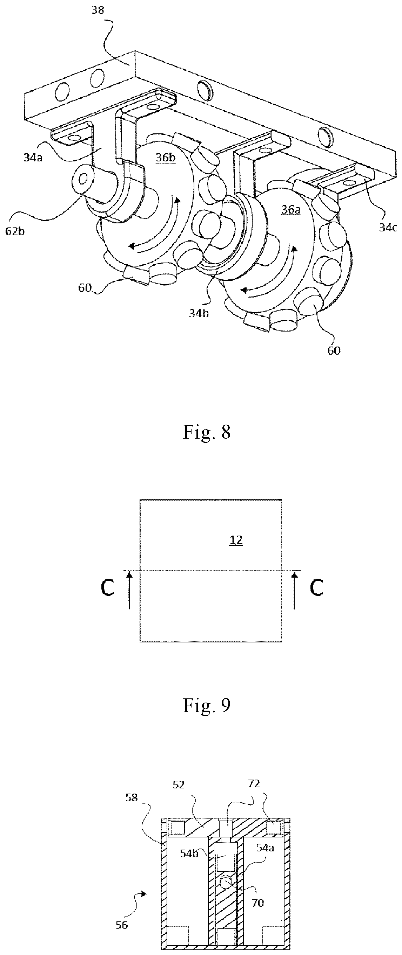

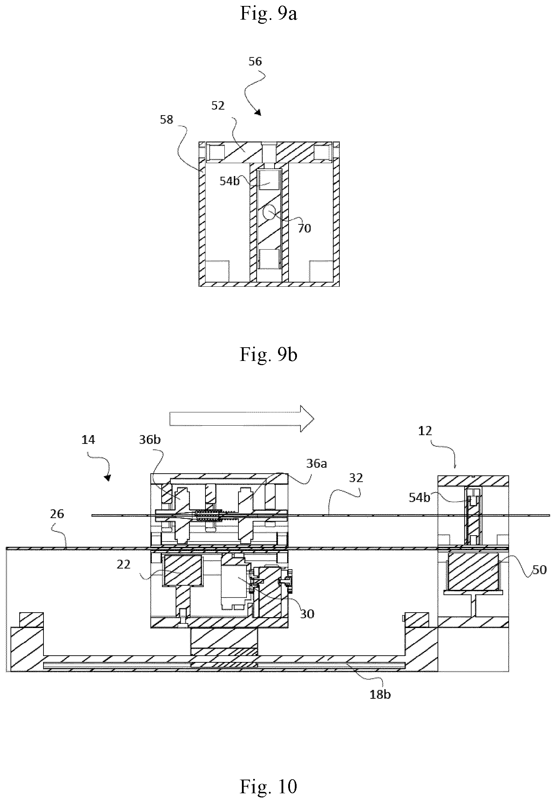

Referring to Figs. 1 and 9a, the second electric magnet 50 is housed by base housing 66 as well, on the reusable non-sterile side. An upper part of robot stationary hand 12 comprises a stationary hand holder 56, which is housed by stationary hand holder housing 58. Stationary hand holder housing 58 comprises a stationary hand roof support 52 which supports stationary hand clamp cylinder 54a. Preferably, Stationary hand holder housing 58 is disposable.

In comparison to the existing art operation operated without surgical robot apparatus 100, an operator must stand near the clinical subject, in place of surgical robot apparatus 100, to manipulate the endo-tool into or out of the clinical subject. Due to many clinical procedures that can require extensive use of x-ray based fluoroscopes, long term exposure to radiation needs to be prevented. Limiting hours of exposure leads to the need for more people on more shifts, which increases operational costs while subjecting operators to undesirable working situations.

Therefore, the embodiments of surgical robot apparatus 100 according to the present disclosure are configured to mimic and achieve the function of the two hands closely to the above-described manner, in a simple and effective way. And very importantly, surgical robot apparatus 100 is configured in a way separating the parts on the sterile side from the non-sterile side. This allows for spatially contactless control/drive of the manipulation of the endo-tool and prevents some expensive components from being contaminated and allows them to be reused from one clinical procedure to another. Furthermore, this allows for surgical robot apparatus 100 to replace the human operator, negating the issues of radiation exposure and extending the time span of each operator operating when they are working remotely. Yet furthermore, this potentially removes the geographical limitations placed on operators who have the expertise on the clinical procedure.

In light of the above, robot moving hand 10 of surgical robot apparatus 100 according to the present disclosure is configured to perform the functions of, 1) holding endo-tool 32 to push or pull endo-tool 32; 2) holding and rotate endo-tool 32.

Referring to Figs. 1~5, robot moving hand 10 includes moving hand manipulator 14 and moving hand actuator 16. In an example embodiment, under spatially contactless control of moving hand actuator 16, moving hand manipulator 14 is configured to maneuver endo-tool 32 to be inserted to or withdrawn from the clinical subject. To achieve such, moving hand actuator 16 comprises at least one magnetic driving gear, moving hand manipulator 14 comprises at least one magnetic induction gear corresponding to the magnetic driving gear, so that magnetic induction gear is spatially contactlessly controlled by magnetic driving gear. In this example embodiment, moving hand manipulator 14 is configured to have a magnetic induction rotating gear 36a and a magnetic induction holding gear 36b, each is respectively supported by a bearing hanger 34c and a bearing hanger 34a, which are attached to a moving hand hanger support 38. Moving hand hanger support 38 is a part of or fixed to a roof of moving hand manipulator housing 15.

In this example embodiment, moving hand actuator 16 comprises two sub-actuators, one the first electric magnet 22, the other a magnetic driving gear 30 driven by actuator motor 24, both of which are controlled by a surgical operator (not shown), preferably by means of remote controls. Magnetic induction rotating gear 36a and magnetic induction holding gear 36b are spatially contactlessly controlled by magnetic driving gear 30 and the first electric magnet 22 respectively to maneuver endo-tool 32 to be inserted to or withdrawn from the clinical subject.

As can be seen in Figs. 1 and 2, a plurality of gear permanent magnets 60 are attached to magnetic induction rotating gear 36a, magnetic induction holding gear 36b, and magnetic driving gear 30. In this example embodiment, the permanent magnets 60 each is a permanent magnetic block in the form of a disc, which is embedded in the outer circumference of magnetic induction rotating gear 36a, magnetic induction holding gear 36b, and magnetic driving gear 30. The plurality of gear permanent magnets 60 each of magnetic induction rotating gear 36a, magnetic induction holding gear 36b, and magnetic driving gear 30 is even-numbered and two adjacent permanent magnet blocks have opposite polarity. Thus magnetic induction rotating gear 36a, magnetic induction holding gear 36b, and magnetic driving gear 30 also refer to as permanent magnetic induction rotating gear 36a, permanent magnetic induction holding gear 36b, and permanent magnetic driving gear 30, respectively. The first electric magnet 22 is controlled by a surgical operator (not shown). The control of the first electric magnet 22 on magnetic induction holding gear 36b is achieved by the magnetic force between the magnetic field of the first electric magnet 22 and gear permanent magnets 60 on magnetic induction holding gear 36b, so keeping magnetic induction holding gear 36b stationary, without the first electric magnet 22 coming into directly or indirectly contact with magnetic induction holding gear 36b. Similarly, the control of magnetic driving gear 30 on magnetic induction rotating gear 36a and/or magnetic induction holding gear 36b is achieved by magnetic force paired up between corresponding gear permanent magnets 60 on magnetic induction rotating gear 36a and/or magnetic induction holding gear 36b and magnetic driving gear 30, thereby driving magnetic induction rotating gear 36a and magnetic induction holding gear 36b to synchronously rotate. Thus, contactless control/drive is achieved in a completely enclosed space, so performing spatially contactless force transmission between inside and outside of the completely enclosed space. There is no need to design a complex structure on base housing 66 and moving hand manipulator housing 15 to install a transmission mechanism and achieve drive by means of contact transmission mechanism between magnetic driving gear 30 and magnetic induction rotating gear 36a and/or magnetic induction holding gear 36b. It completely avoids the entry or leakage of body fluid or contamination during drive process of directly or indirectly contact transmission mechanism. In alternative embodiments, permanent magnetic rings cover on the outer circumference of magnetic induction rotating gear 36a and/or magnetic induction holding gear 36b and magnetic driving gear 30, respectively, instead of gear permanent magnets 60. Each permanent magnetic ring has an even number of permanent magnet segments in the circumferential direction, with two adjacent permanent magnet segments of opposite polarity.

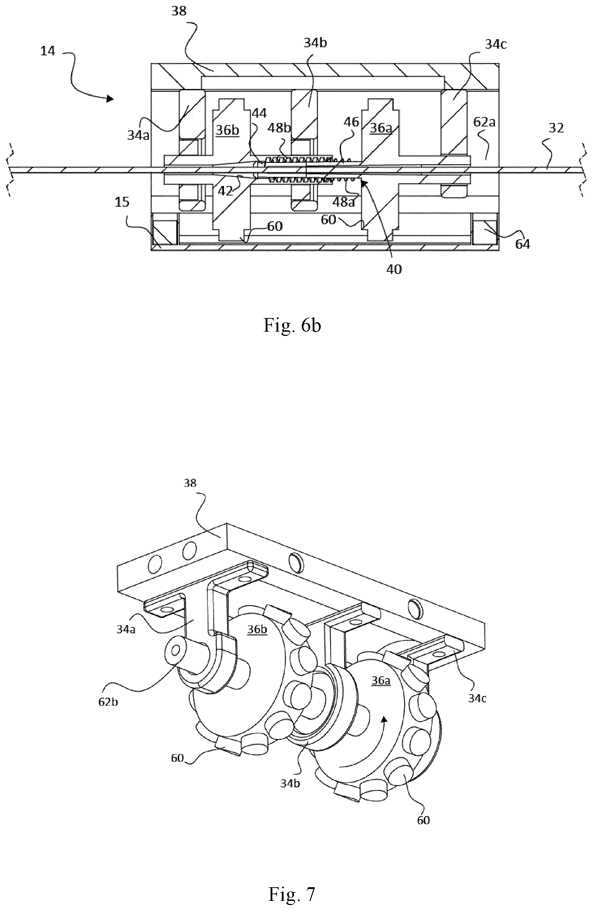

Reference now is made to Figs. 6, 6a, 6b, 7, and 8. Fig. 6 is a top view of the moving hand manipulator 14 of Fig. 5. Fig. 6a is a cross-sectional view of Fig. 6 along B-B, with collet chuck in an open position. Fig. 6b is a cross-sectional view of Fig. 6 along B-B, with collet chuck in a closed position. Fig. 7 is a perspective view of the moving hand manipulator (no manipulator housing 15) in accordance with the present disclosure, illustrating the operation from open to closed of the collet chuck thereof. Fig. 8 is a perspective view of the moving hand manipulator (no manipulator housing 15) in accordance with the present disclosure, showing the rotation of the collet chuck thereof.

Referring to Figs. 6, 6a, 6b, 7, and 8, moving hand manipulator 14 further comprises two cylindrical endo-tool bearings 62a, 62b projecting on the outer sides of magnetic induction rotating gear 36a and magnetic induction holding gear 36b, respectively. The endo-tool 32 is threaded through cylindrical endo-tool bearings 62a, 62b. Cylindrical endo-tool bearings 62a, 62b are supported by bearing hangers 34a, 34b, and 34c. Moving hand manipulator 14 further comprises a collet chuck 40 nestled between magnetic induction rotating gear 36a and magnetic induction holding gear 36b, and supported by bearing hanger 34b.

Collet chuck 40 includes a collet nozzle 42, a collet barrel 48, a collet spindle 46 for coupling with collet barrel 48, and elastic collet tweezer 44. Collet barrel 48 protrudes on the inner side of magnetic induction holding gear 36b. Cylindrical endo-tool bearing 62b and collet barrel 48 are placed on the two opposite outer and inner sides of magnetic induction holding gear 36b. As can be seen in Figs. 6a and 6b, collet barrel 48 has inner threads 48b on one end (towards the right in this example). Collet nozzle 42 is defined in the center of magnetic induction holding gear 36b and extends through cylindrical endo-tool bearing 62b and collet barrel 48 on the opposite sides thereof. Collet nozzle 42 is gradually narrower from the front (right) to the back (left). Collet spindle 46 protrudes on the inner side of magnetic induction rotating gear 36a. Cylindrical endo-tool bearing 62a and collet spindle 46 are placed on the two opposite outer and inner sides of magnetic induction rotating gear 36a. Collet spindle 46 is positioned inside collet barrel 48 coaxially, having outer threads 48a corresponding to the inner threads 48b and configured to be screwed more to collet nozzle 42 (towards the left in this example). Collet spindle 46 further extends and forms collet tweezer 44, which can be engaged in collet nozzle 42. Collet tweezer 44 includes at least two opposite pieces. Endo-tool 32 is threaded through collet barrel 46, collet tweezer 44, and collet nozzle 42. In this example, when magnetic induction rotating gear 36a is turned clockwise, spatially contactlessly controlled by magnetic driving gear 30, while magnetic induction holding gear 36b is stationary, collet spindle 46 is turned more into collet barrel 48, pushing the two or more pieces of collet tweezer 44 more into the gradually narrower collet nozzle 42, forcing them to close upon endo-tool 32, until the two or more pieces of collet tweezer 44 firmly hold onto endo-tool 32, resulting collet chuck 40 to be in a closed position as shown in Fig. 6b. Vice versa, when magnetic induction rotating gear 36a is turned counter clockwise, spatially contactlessly controlled by magnetic driving gear 30, collet spindle 46 is screwed away from collet barrel 48, bringing the two or more pieces of collet tweezer 44 away from collet nozzle 42 into the wider area of collet barrel 48, releasing endo-tool 32, resulting collet chuck 40 to be in an open position as shown in Fig. 6b. In this example, collet spindle 46 and collet barrel 48 are coupled with each other together by use of a Luer connection structure.

Referring to Figs. 7 and 8, with continued reference also made to Figs. 1 and 6b, it should be noted that when it is intended to grab or hold endo-tool 32 or to set collet chuck 40 to from the open to the closed position, magnetic induction holding gear 36b is held still by switching the first electric magnet 22 to an engaged status. As such, when magnetic induction rotating gear 36a is turned clockwise, magnetic induction holding gear 36b does not magnetic induction rotating gear 36a, resulting collet spindle 46 being screwed more into collet barrel 48 until collet chuck 40 being in the closed position. When it is intended to apply a torque, or to rotate endo-tool 32, and when endo-tool 32 is already grabbed by the two or more pieces of collet tweezer 44 or collet chuck 40 is already in the closed position, magnetic induction holding gear 36b is released by switching the first electric magnet 22 to a disengaged status. As a result, the torque can be applied by rotating magnetic induction rotating gear 36a in either direction and magnetic induction holding gear 36b follows the moves of magnetic induction rotating gear 36a in the correspond direction. In alternative embodiments, the torque can be applied by simultaneously rotating magnetic induction rotating gear 36a and magnetic induction holding gear 36b in the same direction.

As such, moving hand manipulator 14 is configured to achieve holding or releasing, pushing or pulling, and torquing/rotating endo-tool 32 in a simple manner.

Reference is now made to Figs. 9, 9a, and 9b. Fig. 9 is a top view of the robot stationary hand 12 in according with the patent disclosure. Fig. 9a is a cross-sectional view of Fig. 9 along C-C, with the robot stationary hand in an open position. Fig. 9b is a cross-sectional view of Fig. 9 along C-C, with the robot stationary hand in a closed position.

As shown in Fig. 9, 9a, and 9b, robot stationary hand 12 comprises the second electric magnet 50 and stationary hand holder 56. The second electric magnet 50 is on the non-sterile side and housed by base housing 66. Stationary hand holder 56 is on the sterile side and housed by stationary hand holder housing 58. Stationary hand holder 56 comprises a stationary hand cylinder 54a, a stationary hand clamp 70 sliding in stationary hand cylinder 54a, and a stationary hand magnetic element 54b fixed on stationary hand clamp 70, with stationary hand cylinder 54a supported by stationary hand roof support 52 via fasteners 72.

Robot stationary hand 12 is operated by switching the second electric magnet 50 to an engaged status or a disengaged status with the electromagnetic force of the second electric magnet 50 attracting or releasing stationary hand magnetic element 54b. As a result, stationary hand clamp 70 slidably moves in stationary hand cylinder 54a to close to endo-tool 32 and hold endo-tool 32, or to remove from endo-tool 32 to release endo-tool 32, respectively. See Fig. 9a and 9b, where stationary hand clamp 70 is in an open position and in a closed position, releasing or holding endo-tool 32 threaded through stationary hand clamp 70, respectively.

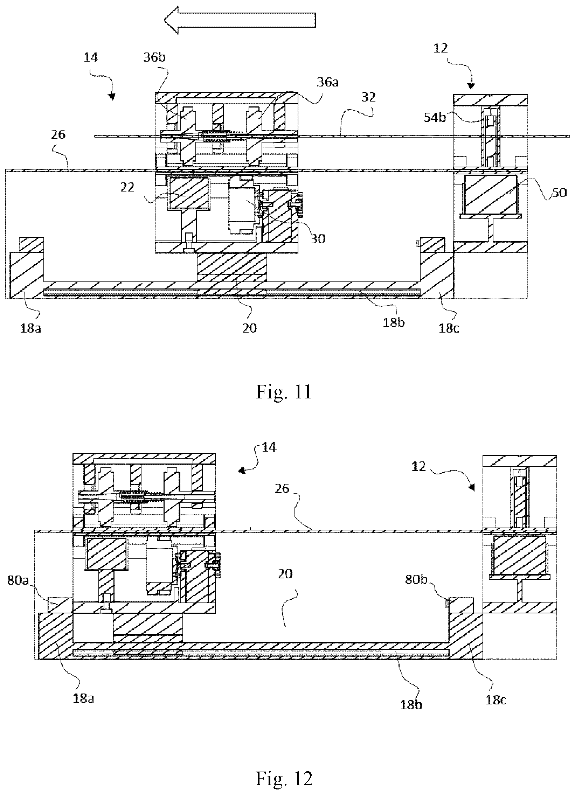

Reference is now made to Figs. 10 and 11. Fig. 10 is another view of Fig. 4, with an endo-tool 32 added and held by moving hand manipulator 14 for inserting the endo-tool 32 into a clinical subject. Fig. 12 is yet another view of Fig. 4, with an endo-tool 32 added and held by the moving hand manipulator 14 for removing the endo-tool 32 from a clinical subject, all in accordance with the present disclosure.

Referring to Figs. 10 and 11, with continued reference made to Fig. 6b, when collet chuck 40 is at its closed position, endo-tool 32 is held or grabbed by collet chuck 40, and by the moving of actuator platform 20 along track 18b from backend track stand 18a to frontend track stand 18c, moving hand actuator 16 on actuator platform 20 synchronizes the movement of the moving hand manipulator 14 by a connecting structure between moving hand manipulator 14 and moving hand actuator 16, to drive the entire moving hand manipulator 14 traversing towards the clinical subject, endo-tool 32 is gradually pushed into the clinical subject for a distance of roughly between backend track stand 18a and frontend track stand 18c.

After actuator platform 20 reaches frontend track stand 18c, one segment of endo-tool 32 is already pushed into the clinical subject, resulting moving hand manipulator 14 being moved closer to robot stationary hand 12. At this moment, in a similar fashion of how an existing manual operation does, robot stationary hand 12 closes stationary hand magnetic element 54b by engaging the second electric magnet 50, holding and stabilizing endo-tool 32 not moving. At the meanwhile, moving hand manipulator 14 is configured to set collet chuck 40 to be in its open position by the spatially contactless control/drive of the first electric magnet 22 and magnetic driving gear 30, releasing endo-tool 32. Subsequently, by the moving of actuator platform 20 along track 18b from frontend track stand 18c to back to backend track stand 18a, moving hand actuator 16 on actuator platform 20 synchronizes the movement of the moving hand manipulator 14 by connecting structure between moving hand manipulator 14 and moving hand actuator 16, to drive the entire moving hand manipulator 14 traversing away from the clinical subject. After actuator platform 20 reaches back to backend track stand 18a, surgical robot apparatus 100 is ready for another cycle of inserting or pushing the next segment of endo-tool 32 into the clinical subject. Then stationary hand holder 56 is in the open position, releasing endo-tool at that point as well and collet chuck 40 clamps down again on endo-tool 32.

Similar coordination among moving hand manipulator 14 and robot stationary hand 12 applies during the process of removing endo-tool 32 from the clinical subject. That is, during the time that collet chuck 40 is in a closed position to push, pull or torque/rotate endo-tool 32, robot stationary hand 12 is at the open position where stationary hand magnetic element 54b is released, thereby coming loose endo-tool 32. During the time moving hand manipulator 14 is transferring from one segment of endo-tool 32 to another, collet chuck 40 is in an open position and releases endo-tool 32, and robot stationary hand 12 is at the closed position where stationary hand magnetic element 54b is attracted, thereby holding endo-tool 32, stopping endo-tool 32 from moving and affecting the clinical subject while moving hand manipulator 14 is in transition.

In alternative embodiments, stationary hand holder 56 of robot stationary hand 12 loosely supports endo-tool 32 to allow endo-tool 32 movable relative to stationary hand holder 56. Thus, robot moving hand 10 with endo-tool 32 together can move relative to stationary hand holder 56 in the single direction, thereby achieving pushing or pulling, and torquing/rotating of endo-tool 32, as long as track 18b is long enough and robot moving hand 10 does not need to move along track 18b back and forth. That is, stationary hand holder 56 supports only endo-tool 32 from tortuosity so that it straightens up.

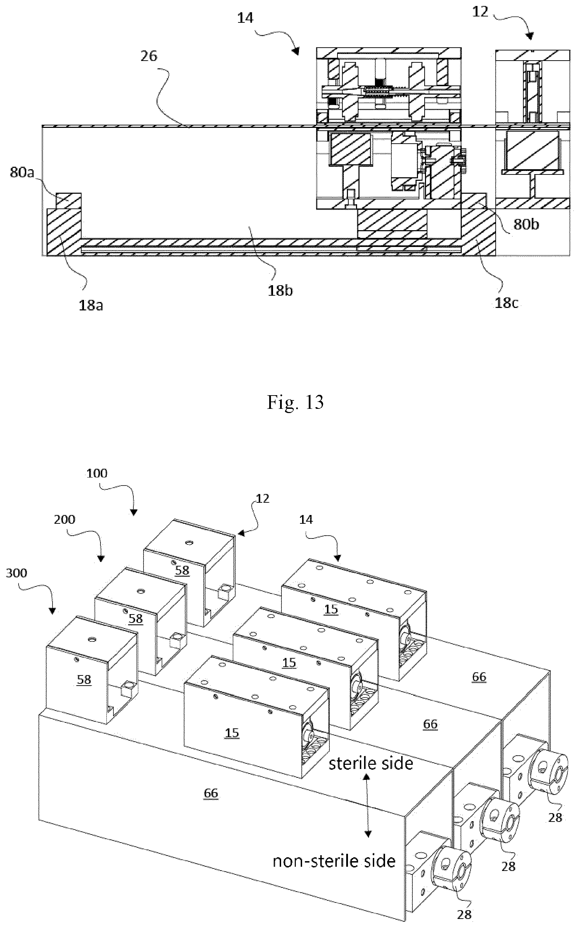

Reference is now made to Figs. 12 and 13. Fig. 12 is yet another view of Fig. 4, with the robot moving hand in an initial position of sensor triggers. Fig. 12 is yet another view of Fig. 4, with the robot moving hand in an ultimate position of sensor triggers, all in accordance with the present disclosure.

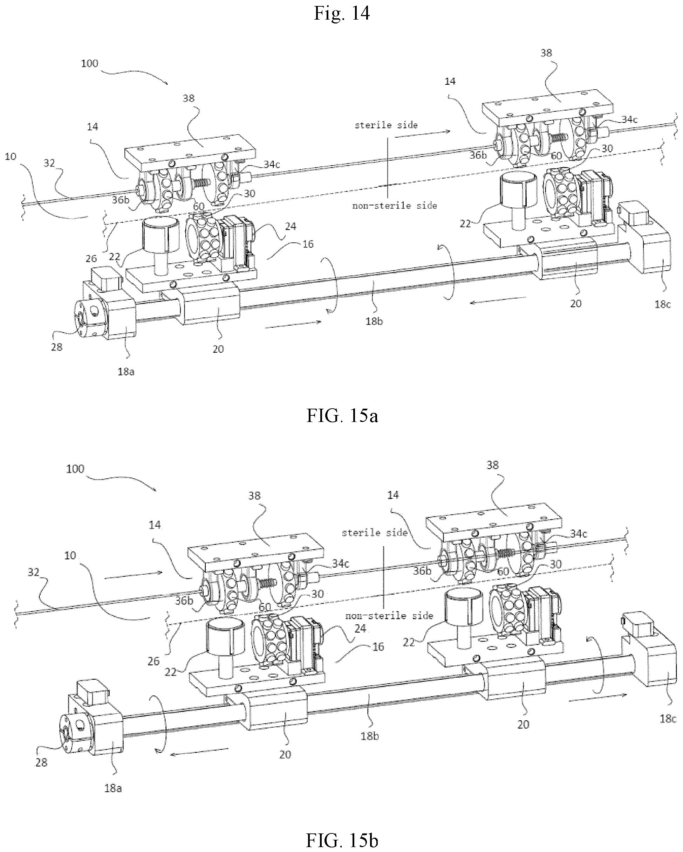

Referring to Figs. 13 and 14, in an example embodiment, the operation and sequence of movement of elements of surgical robot apparatus 100 are fully or partially controlled by automatic controls, either via wired or wireless controls. Surgical robot apparatus 100 therefore further comprise sensors to facilitate the automatic controls.

In an example embodiment, surgical robot apparatus 100 further comprises a backend position sensor 80a and a frontend position sensor 80b, which are configured to sense the presence of actuator platform 20 reaching backend track stand 18a or frontend track stand 18c, respectively. Backend position sensor 80a and frontend position sensor 80b are respectively supported to backend track stand 18a and frontend track stand 18c. Backend position sensor 80a and frontend position sensor 80b may be the kind of motion sensors based on passive infrared (PIR), ultrasonic, microwave or to myographic detecting energies to measure the distance of actuator platform 20 to backend track stand 18a and frontend track stand 18c, and therefore providing information on how far endo-tool 32 has traveled into the clinical subject. Backend position sensor 80a and frontend position sensor 80b may be the kind of touch sensors such as pressure sensor or light sensor, which provide information when actuator platform 20 reaches backend track stand 18a or frontend track stand 18c.

As shown in Fig. 12, when backend position sensor 80a senses actuator platform 20 reaches backend track stand 18a, surgical robot apparatus 100 in according with the present disclosure is ready for another cycle of inserting or pushing the next segment of endo-tool 32 into the clinical subject. At this moment, the first electric magnet 22 and magnetic driving gear 30 spatially contactlessly controls magnetic induction rotating gear 36a and magnetic induction holding gear 36b, thereby setting collet chuck 40 to be at its closed position, holding endo-tool 32. Actuator platform 20 is controlled to move robot moving hand 10 towards frontend track stand 18c. When the entire robot moving hand 10 traverses towards the clinical subject by the moving of actuator platform 20 along track 18b from backend track stand 18a to frontend track stand 18c, endo-tool 32 is gradually pushed into the clinical subject for a distance of roughly between backend track stand 18a and frontend track stand 18c.

As shown in Fig. 13, when frontend position sensor 80b senses actuator platform 20 reaches frontend track stand 18c, one segment of endo-tool 32 is already pushed into the clinical subject, the second electric magnet 50 is then controlled to close stationary hand magnetic element 54b by pulling stationary hand magnetic element 54b to hold and stabilize endo-tool 32 not moving. At the meanwhile, magnetic driving gear 30 is controlled to set collet chuck 40 to be at its open position, releasing endo-tool 32. Subsequently, actuator platform 20 is controlled to move robot moving hand 10 to traverse away from the clinical subject by a long track 18b from frontend track stand 18c to back to backend track stand 18a.

Surgical robot apparatus 100 in according with the present disclosure may also comprise other sensors enabling automatic control to control the coordination among moving hand manipulator 14 and robot stationary hand 12. For example, during the process of removing endo-tool 32 from the clinical subject, that is, during the time that collet chuck 40 is in a closed position to push, pull or torque/rotate endo-tool 32, robot stationary hand 12 is at the opened position where stationary hand magnetic element 54b is released, thereby coming loose endo-tool 32. During the time moving hand manipulator 14 is transferring from one segment of endo-tool 32 to another, collet chuck 40 is in an open position and release endo-tool 32, and robot stationary hand 12 is at the closed position where stationary hand magnetic element 54b is attracted, thereby holding endo-tool 32, stopping endo-tool 32 from moving and affecting the clinical subject while moving hand manipulator 14 is in transition. It prevents to affect the clinical subject

Fig. 14 is an assembly view of three of the surgical robot apparatus 100 in Fig. 5 showing an embodiment with multiple pairs of robot moving hands and robot stationary hands for the operation of multiple endo-tools on a clinical subject in accordance with the present disclosure.

Due to the simplistic configuration of surgical robot apparatus 100, there is enough space clinically to arrange multiple pairs of similar surgical robot apparatuses, such as surgical robot apparatuses 200 and 300 on a catheter bed as shown in Fig. 14. The configuration of surgical robot apparatuses 200 and 300 can be the same or similar surgical to surgical robot apparatus 100. As a result, multiple endo-tools, such as catheters carrying surgical devices, implantable devices and interventional devices, diagnostic sensors, medicine, etc. can be inserted into or removed from the clinical subject, all during the same clinical procedure.

Although three surgical robotic apparatuses are illustrated in FIG. 14, this cannot be understood as a limitation on the scope of the present disclosure. In alternative embodiments, number of surgical robot apparatus in according with the present disclosure can be determined by clinical procedures.

It can be appreciated that the operation of surgical robot apparatus 100 can also be perform by any combination of human operation and automation. For example, the operation of magnetic induction holding gear 36b and magnetic induction rotating gear 36a can be done remotely by a human holding a remote controller while the movement of actuator platform 20 can be done automatically, without magnetic driving gear 30 and the first electric magnet 22. For another example, only robot moving hand 10, without robot stationary hand 12, is configured to achieve pushing or pulling, and torquing/rotating endo-tool 32. At this time, for support purposes, clips may be secured to the base housing 66 to hold and support endo-tool 32 from tortuosity. When robot moving hand 10 is configured to push, pull or torque/rotate endo-tool 32, endo-tool 32 can normally move and rotate whilst the clips don't fully release endo-tool 32. For yet another example, robot stationary hand 12 includes only two opposite frictional rollers but the second electric magnet 50. Then, endo-tool 32 is placed between two opposite frictional rollers but is free to be pushed or pulled and/or torqued/rotated, actuator platform 20 carrying robot moving hand 10 moves along the single direction to achieve pushing, pulling or torquing/rotating of endo-tool 32, without back and forth, as long as track 18b is long enough. All such alternative embodiments are within the scope of the present disclosure.

In alternative embodiments, it is achieved that spatially contactless controls magnetic induction rotating gear 36a to rotate clockwise or counterclockwise to rotate collet spindle 46 into or out of collet barrel 48 respectively by another magnetic driving gear being driven by another actuator motor (as magnetic driving gear 30 being driven actuator motor 24), whereas magnetic driving gear 30 is configured to only drive magnetic induction rotating gear 36a in spatially contactless controlling manner, thereby magnetic induction holding gear 36b follows magnetic induction rotating gear 36a simultaneously rotating in the same direction. In other embodiments, collet chuck 40 is spatially contactlessly controlled by the first and second magnetic driving gears as magnetic driving gear 30, instead of one magnetic driving gear 30 and one the first electric magnet 22 respectively in this embodiment. For example, the first magnetic driving gear spatially contactlessly controls magnetic induction holding gear 36b to rotate one of clockwise and counter clockwise, while the second magnetic driving gear spatially contactlessly controls magnetic induction rotating gear 36a to rotate the other of clockwise and counter clockwise, thereby collet chuck 40 is at the closed position to achieve holding of endo-tool 32. This enables fast holding for improved efficiency. Then the first and second magnetic driving gears are rotated simultaneously, thereby spatially contactlessly controlling magnetic induction holding gear 36b and magnetic induction rotating gear 36a to rotate in the same clockwise direction. Of course, only one of the first and second magnetic driving gears may spatially contactlessly controls corresponding one of magnetic induction holding gear 36b and magnetic induction rotating gear 36a, thereby collet chuck 40 is as well at the closed position and at the open position except that closing and opening becomes slower. All such alternative embodiments are within the scope of the present disclosure.

In addition, manipulations of the right and left hands can be exchanged during manual operation depending on the operator's practice. For example, assuming the operator stands with his/her back facing the reader of Fig. 1, during the insertion of endo-tool 32, the operator lets the left hand loosely support endo-tool 32. At the same time, the operator uses the right hand (sometimes both hands) to push, pull or torque/rotate endo-tool 32. When one segment of endo-tool 32 is pushed into the clinical subject, resulting in the right hand being moved closer to the left hand, the operator would need to close the left hand, holding endo-tool 32 to not move, and at the same time, move release and move the right hand further down to the right to allow if to be ready to push and/or rotate the next segment of endo-tool 32 into the clinical subject. Similarly, herein these include a clinical subject to the far left hand side of the diagram of Fig. 1. And robot stationary hand 12 is located on the left side of robot moving hand 10 and is secured between the clinical subject and robot moving hand 10.

In alternative embodiments, another track 18b is positioned in base housing 66 and supported by another backend track stand 18a and another frontend track stand 18c of base housing 66, and another actuator platform 20 is slidably secured on another track 18b for carrying the second electric magnet 50 of robot stationary hand 12. Herein robot stationary hand 12 is located away from the clinical subject, and that is, robot moving hand 10 is secured between robot stationary hand 12 and the clinical subject. The stationary hand holder housing 58 is slidably and detachably secured to the base housing 66, as that of manipulator housing 15. When collet chuck 40 and stationary hand holder 56 are in the closed position by manipulations of moving hand manipulator 14 and the second electric magnet 50, respectively, robot moving hand 10 and robot stationary hand 12 holds endo-tool 32 at the same time. Thus, actuator platform 20 for carrying moving hand actuator 16 is moved along track 18b from backend track stand 18a to frontend track stand 18c, and another actuator platform 20 for carrying the second electric magnet 50 is moved along another track 18b from another backend track stand 18a to another frontend track stand 18c, thereby moving hand manipulator 14 and stationary hand holder 56 together holding endo-tool 32 and moving towards the clinical subject. After actuator platform 20 reaches frontend track stand 18c, one segment of endo-tool 32 is already pushed into the clinical subject. Then actuator platform 20 and another actuator platform 20 stop movement, collet chuck 40 shifts to the open position while stationary hand holder 56 maintains in the closed position. Actuator platform 20 carries moving hand manipulator 14 away from frontend track stand 18c to back to backend track stand 18a. When actuator platform 20 moves a certain distance, allowing for collet chuck 40 and stationary hand holder 56 in the closed position again. Actuator platform 20 for carrying moving hand actuator 16 is moved along track 18b from backend track stand 18a to frontend track stand 18c, and another actuator platform 20 for carrying the second electric magnet 50 is continuously moved along another track 18b towards another frontend track stand 18c. Moving hand manipulator 14 and stationary hand holder 56 together holds endo-tool 32 and further moves into the clinical subject. It is repeated until endo-tool 32 is moved in place. Preferably, another track 18b is longer than track 18b. When endo-tool 32 is to be withdrawn from the clinical subject, the above manipulations basically and reversibly proceed with even some minor changes. For example, another robot stationary hand 12 is added between robot moving hand 10 and the clinical subject in the alternative embodiment and includes two opposite rows of frictional rollers without the second electric magnet 50. Its specific operation is referred to the above. In addition, there also have a mounting structure for supporting hemostasis valve between robot stationary hand 12 and another robot stationary hand 12. With requirements, hemostasis valve, which is supported on one mounting structure away from the clinical subject, can be supported on another mounting structure close to the clinical subject. Furthermore, a rapid exchange unit can be installed on robot stationary hand 12 away from the clinical subject and is configured to move rapid-exchange catheter.

In the above alternative embodiment, there may also only one track 18b located in base housing 66. That is, actuator platform 20 and another actuator platform 20 are secured on the same track 18b, and are driven by two different motors to move, are not driven by one track motor coupler 28 or a pair of track motor couplers 28, thereby performing the above manipulations of endo-tool 32.

In the other embodiment, robot stationary hand 12 may also be replaced by another robot moving hand 10. Thus, surgical robot apparatus includes two robot moving hands 10 linearly sliding on track towards or away from the clinical subject, thereby pushing or pulling, and torquing/rotating endo-tool 32. Two robot moving hands 10 can move one-way or back and forth either on one track or on two different track and can be driven by one motor or two motors.

Referring to Figs. 15a and 15b, track 18b is a rod of screw assembly driven by track motor coupler 28, which is provided with two segments of threads with opposite thread directions, as indicated by the arrows in the diagrams. Two nuts of screw assembly are respectively secured on two actuator platforms 20 each for carrying robot moving hand 10. At the beginning, collet chuck 40 of the left robot moving hand 10 is in the closed position while collet chuck 40 of the right robot moving hand 10 is in the open position, track motor coupler 28 drives rod of screw assembly rotate in one of clockwise and counter clockwise, and two robot moving hands 10 are closed to each other, thereby moving of endo-tool 32 towards the clinical subject, as shown in Fig. 15a. When two robot moving hands 10 are moved to the limit in opposite directions, collet chuck 40 of the left robot moving hand 10 shifts in the open position while collet chuck 40 of the right robot moving hand 10 shifts in the closed position. Track motor coupler 28 drives rod of screw assembly rotate in the other of clockwise and counter clockwise, and two robot moving hands 10 are moved away from each other, thereby continuously moving of endo-tool 32 towards the clinical subject, as shown in Fig. 15b. There is an achievement of continuous movement of endo-tool 32 into the clinical subject. When endo-tool 32 is to be withdrawn from the clinical subject, the above manipulations reversibly proceed. In alternative embodiment, the screw assembly includes two rods driven two track motor couplers 28, respectively. Two rods of the screw assembly have threads with opposite thread directions. Furthermore, there may be allowing for moving back and forth of robot moving hand 10 close to the clinical subject, and for moving one-way of robot moving hand 10 away from the clinical subject, thereby performing movement of endo-tool 32. Similarly, when endo-tool 32 is to be withdrawn from the clinical subject, the above manipulations reversibly proceed as well.

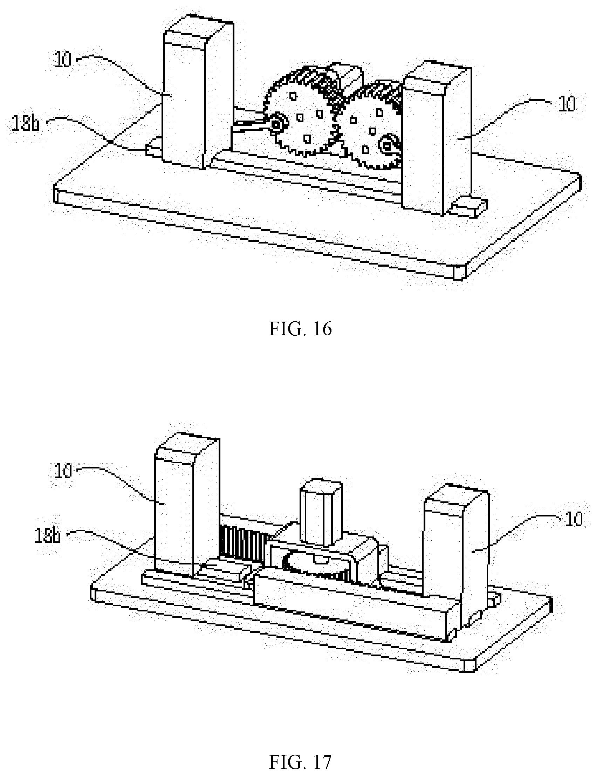

Referring to Figs. 16 and 17, which illustrate the movement back and forth of coordinated two robot moving hands 10 driven by gear linkage transfer system and rack and pinion of transfer system, respectively, for performing continuous inserting of endo-tool in one direction. Two robot moving hands 10 are driven by one motor in shown in the above figures, but two robot moving hands 10 can be respectively driven by two different motors in alternative embodiment. In addition, there may be allowing for movement back and forth of robot moving hand 10 close to the clinical subject, and for one-way movement of robot moving hand 10 away from the clinical subject, thereby performing continuous inserting movement of endo-tool 32. Similarly, when endo-tool 32 is to be withdrawn from the clinical subject, the above manipulations reversibly proceed as well.

For connection, displacement driving and rotation driving between moving hand actuator 16 and moving hand manipulator 14, and holding between the second electric magnet 50 and stationary hand holder 56, in addition to the above permanent magnet/electromagnetic driving for spatially contactless control/drives, it is also achieved by other spatially contactless control/drives such as electromagnetic induction, electric field coupling, direct current resonance (DC resonant), and etc. It noted that spatially contactless control/drives in the present disclosure refers to control/drive being achieved spatially without contact and does not refer to control/drive through an air medium.

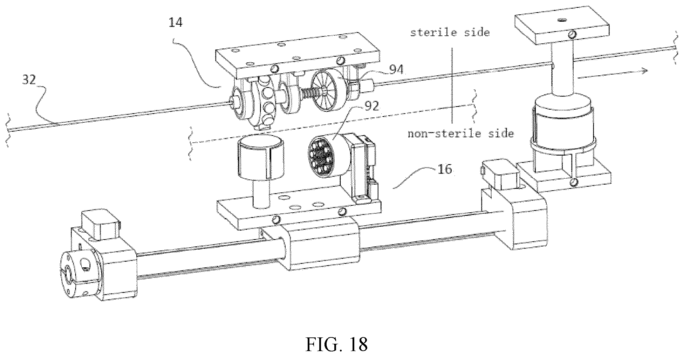

The following is illustrated as an example of an electromagnetically induced spatially contactless control/drive between moving hand actuator 16 and moving hand manipulator 14, but it is equally applicable to a spatially contactless control/drive between the second electric magnet 50 and stationary hand holder 56. As shown in Fig. 18, in electromagnetically induced spatially contactless control/drive manner, moving hand actuator 16 includes stator winding 92, and moving hand manipulator 14 correspondingly comprises a ferromagnetic rotor 94. When stator winding 92 is energized, a varying magnetic field is generated and acts on a ferromagnetic rotor 94. That is, a magneto-electric rotational torque is formed to allow ferromagnetic rotor 94 to rotate endo-tool 32.

For DC resonant spatially contactless control/drive manner, perhaps the principle of spatially contactless control/drive of the stator and rotor in a resonant DC motor can be as reference.

As can be seen from the above, spatially contactless control/drive in according with the present disclosure is achieved by contactless force, i.e., electric field force, magnetic field force and other kinds of field force, without medium, and so it is possible to do so in a vacuum. Thus, any manner that utilizes the force of any field on the object into its field to achieve spatially contactless control/drive, is within the scope and spirit of the present disclosure.

The surgical robot apparatus 100 in according with the present disclosure performs spatially contactless control/drive with expensive components such as mechanical drive gears and motors placed on the non-sterile side in a completely enclosed space, thereby realizing sterile isolation. There is no need to design a complex structure on base housing 66 and moving hand manipulator housing 15 to install a transmission mechanism and achieve drive by means of contact transmission mechanism between magnetic driving gear 30 and magnetic induction rotating gear 36a and/or magnetic induction holding gear 36b. It completely avoids the entry or leakage of body fluid or contamination during controlling/driving process of directly or indirectly contact control/drive mechanism and prevents some expensive components such as mechanical drive gears and motors being contaminated, and provides protection against them to the maximum extent.

The above-mentioned embodiments only express limited implementation methods of the present disclosure, and their descriptions are relatively specific and detailed, but they cannot be understood as limiting the scope of the present disclosure. It should be pointed out that for ordinary technicians in this field, without departing from the concept of the present disclosure, several deformations and improvements or deteriorations can be made. For example, robot moving hand 10 and its deformation, robot stationary hand 12 and its deformation or even rapid exchange unit and mounting structure for hemostasis valve, etc. can be set up multiple or formed into any combination, so that several endo-tools can move and/or rotate coordinately. For yet example, synchronized movement, synchronized turning/rotation can also be unsynchronized, as long as it is a controlled movement. These all belong to the scope of the present disclosure. Therefore, the scope of the present disclosure shall be based on the claims.

## Claims

1. A surgical robot apparatus comprising a robot moving hand configured to move towards or away from a clinical subject, the robot moving hand comprises a moving hand actuator and a moving hand manipulator, the moving hand actuator and the moving hand manipulator are respectively located on a non-sterile side and a sterile side, which are isolated from each other, the moving hand manipulator is spatially contactlessly controlled by the moving hand actuator to move and/or rotate, thereby the moving hand manipulator is configured to maneuver an endo-tool to be inserted into or withdrawn from a clinical subject.

2. The surgical robot apparatus according to claim 1, wherein the moving hand manipulator is spatially contactlessly controlled by the moving hand actuator to move and/or rotate by means of magnetic driving.

3. The surgical robot apparatus according to claim 1, wherein the moving hand manipulator is spatially contactlessly controlled by the moving hand actuator to move and/or rotate by means of electromagnetic induction driving.

4. The surgical robot apparatus according to claim 1, wherein the moving hand manipulator is spatially contactlessly controlled by the moving hand actuator to move and/or rotate by means of electric field coupling driving.

5. The surgical robot apparatus according to claim 1, wherein the moving hand manipulator is spatially contactlessly controlled by the moving hand actuator to move and/or rotate by means of direct current resonance driving.

6. The surgical robot apparatus according to claim 1, wherein the moving hand actuator comprises at least one magnetic driving gear, the moving hand manipulator comprises at least one magnetic induction gear corresponding to the magnetic driving gear, the magnetic induction gear is spatially contactlessly controlled by the magnetic driving gear.

7. The surgical robot apparatus according to claim 1, wherein the moving hand manipulator comprises a permanent magnetic induction rotating gear and a permanent magnetic induction holding gear, the moving hand actuator comprises a permanent magnetic driving gear and the first electric magnet, the permanent magnetic induction holding gear is spatially contactlessly controlled to not move by the first electric magnet, the permanent magnetic induction rotating gear is spatially contactlessly controlled to rotate by the permanent magnetic driving gear.

8. The surgical robot apparatus according to claim 7, wherein the surgical robot apparatus further comprises a collet chuck nestled between the permanent magnetic induction rotating gear and the permanent magnetic induction holding gear, the collet chuck is configured for an endo-tool threaded therethrough, when the permanent magnetic induction rotating gear and the permanent magnetic induction holding gear rotate relative to each other, the collet chuck is in a closed position to hold the endo-tool or the collet chuck is in an open position to release the endo-tool.

9. The surgical robot apparatus according to claim 8, wherein the collet chuck comprises a collet barrel and a collet spindle for coaxially positioned inside the collet barrel, the collet barrel has inner threads on one end and a collet nozzle on the other end, the collet spindle has outer threads coupled with the inner threads and a collet tweezer corresponding to the elastic collet nozzle, when the collet spindle is threaded more to the other end of the collet barrel and positioned inside the collet barrel, the collet tweezer is pushed more into the collet nozzle, thereby forcing it elastically close upon the endo-tool and hold onto an endo-tool, or when the collet spindle is threaded away from the other end of the collet barrel and withdraws from the collet barrel, the collet tweezer is pulled out of the collet nozzle, the collet tweezer is elastically restored and releases the endo-tool.

10. The surgical robot apparatus according to claim 9, **characterized in that** the collet barrel protrudes from one of the permanent magnetic induction rotating gear and the permanent magnetic induction holding gear, the collet spindle is formed on the other of the permanent magnetic induction rotating gear and the permanent magnetic induction holding gear.

11. The surgical robot apparatus according to claim 9, wherein the collet tweezer comprises a plurality of pieces.

12. The surgical robot apparatus according to claim 1, wherein the moving hand manipulator comprises a permanent magnetic induction rotating gear, a permanent magnetic induction holding gear and a collet chuck, when the permanent magnetic induction holding gear is stationary to not move and the permanent magnetic induction rotating gear rotates relative to the permanent magnetic induction holding gear, the collet chuck is in a closed position to hold the endo-tool and in an open position to release an endo-tool.

13. The surgical robot apparatus according to claim 12, wherein when the collet chuck is in a closed position to hold an endo-tool and the permanent magnetic induction holding gear is released, the permanent magnetic induction rotating gear and the permanent magnetic induction holding gear synchronously rotate together with the endo-tool.

14. The surgical robot apparatus according to claim 8 or 12, wherein the surgical robot apparatus further comprises a track, when the moving hand actuator travels along the track, the moving hand manipulator is moved by spatially contactless control of the moving hand actuator.

15. The surgical robot apparatus according to claim 14, wherein the moving hand actuator and the moving hand manipulator are each provided with magnetic components, magnetic attraction of the magnetic components is configured to engage the moving hand manipulator with the moving hand actuator together.

16. The surgical robot apparatus according to claim 15, wherein when the collet chuck is in a closed position to hold an endo-tool and the permanent magnetic induction holding gear is released, the moving hand actuator travels along the track, the moving hand manipulator synchronously move together with the moving hand actuator by magnetic attraction of the magnetic components of the moving hand actuator and the moving hand manipulator, thereby robot moving hand pushing the endo-tool towards or withdrawing the endo-tool from a clinical subject along the track.

17. The surgical robot apparatus according to claim 8 or 12, wherein the moving hand manipulator further comprises two cylindrical bearings respectively projecting on the permanent magnetic induction rotating gear and the permanent magnetic induction holding gear, the two cylindrical bearings are respectively supported by two bearing hangers, the collet chuck is supported by another bearing hanger between the two bearing hangers.

18. The surgical robot apparatus according to claim 17, wherein the moving hand actuator is housed in a base housing, which is on the non-sterile side, the moving hand manipulator is housed in manipulator housing, which is on the sterile side, the manipulator housing is detachably mounted on the base housing and linearly move relative to the base housing.

19. The surgical robot apparatus according to claim 18, wherein a sterile barrier is placed between the sterile side and the non-sterile side and configured to separate the sterile side from the non-sterile side, the base housing stays on the non-sterile side of the sterile barrier.

20. The surgical robot apparatus according to claim 1, wherein the moving hand manipulator comprises a permanent magnetic induction rotating gear and a permanent magnetic induction holding gear, the moving hand actuator comprises the first permanent magnetic driving gear and the second permanent magnetic driving gear, the permanent magnetic induction holding gear and the permanent magnetic induction rotating gear are spatially contactlessly controlled to rotate by the first permanent magnetic driving gear and the second permanent magnetic driving gear, respectively.

21. The surgical robot apparatus according to claim 1, wherein the surgical robot apparatus further comprises a robot stationary hand, the robot stationary hand comprises a support member on the sterile side for supporting the endo-tool.

22. The surgical robot apparatus according to claim 1, wherein the surgical robot apparatus further comprises a robot stationary hand, the robot stationary hand comprises a stationary hand holder on the sterile side and a controller on the non-sterile side, the stationary hand holder is spatially contactlessly controlled by the controller to hold or release the endo-tool.

23. The surgical robot apparatus according to claim 22, wherein the robot stationary hand is stationary to not move between the robot moving hand and a clinical subject, when the robot stationary hand releases the endo-tool, the moving hand manipulator holds and maneuvers the endo-tool to be inserted into or withdrawn from a clinical subject.

24. The surgical robot apparatus according to claim 22, wherein the robot stationary hand is stationary to not move between the robot moving hand and a clinical subject, when the robot stationary hand releases the endo-tool, when the robot stationary hand holds the endo-tool, the moving hand manipulator releases the endo-tool and moves towards or away from a clinical subject.

25. The surgical robot apparatus according to claim 22, wherein the stationary hand holder is spatially contactlessly controlled by the controller to hold or release by magnetic means.

26. The surgical robot apparatus according to claim 25, wherein the controller is a second electric magnet.

27. The surgical robot apparatus according to claim 26, wherein the stationary hand holder comprises a stationary hand cylinder, a stationary hand clamp slidably placed in the stationary hand cylinder, and a stationary hand magnetic element fixed on the stationary hand clamp, the stationary hand magnetic element is spatially contactlessly controlled by the second electric magnet to be held or released, thereby the stationary hand clamp sliding in the stationary hand cylinder close to and holding the endo-tool or away from the endo-tool and releasing endo-tool.

28. The surgical robot apparatus according to claim 22, wherein the robot moving hand is placed between the robot stationary hand and a clinical subject, when the robot stationary hand and the robot moving hand move together with the endo-tool into the clinical subject or withdraw together with the endo-tool from the clinical subject a predetermined distance, after the robot moving hand moves towards or away from the robot stationary hand a certain distance, the robot stationary hand and the robot moving hand move again together with the endo-tool into a clinical subject or withdraw together with the endo-tool from the clinical subject a predetermined distance.

29. The surgical robot apparatus according to claim 1, wherein the surgical robot apparatus further comprises a robot stationary hand between the robot moving hand and a clinical subject, the robot stationary hand comprises two opposite frictional rollers, which oppositely hold an endo-tool, when the moving hand manipulator holds and maneuvers an endo-tool to be inserted into or withdrawn from a clinical subject, the endo-tool is free to move relative to the frictional rollers.

30. The surgical robot apparatus according to claim 1, wherein the surgical robot apparatus further comprises at least two position sensors, which are configured to sense the presence of the robot moving hand reaching the distal maximum position or the proximal maximum position.

31. A method of operating a surgical robot apparatus, the surgical robot apparatus having a robot moving hand and a moving hand actuator and a moving hand manipulator, the operating method comprising:
providing the moving hand manipulator being placed on a sterile side and the moving hand actuator to be placed on anon-sterile side apart from the sterile side; and
providing the moving hand manipulator to move and/or rotate by spatially contactless controlling of the moving hand actuator, and thereby the moving hand manipulator together with an endo-tool being inserted into a clinical subject or withdrawn from a clinical subject.

32. The method of operating the surgical robot apparatus according to claim 31, wherein the moving hand manipulator is spatially contactlessly controlled by the moving hand actuator to move and/or rotate by means of magnetic driving.

33. The method of operating the surgical robot apparatus according to claim 31, wherein the moving hand manipulator is spatially contactlessly controlled by the moving hand actuator to move and/or rotate by means of electromagnetic induction driving.

34. The method of operating the surgical robot apparatus according to claim 31, wherein the moving hand manipulator is spatially contactlessly controlled by the moving hand actuator to move and/or rotate by means of electric field coupling driving.

35. The method of operating the surgical robot apparatus according to claim 31, wherein the moving hand manipulator is spatially contactlessly controlled by the moving hand actuator to move and/or rotate by means of direct current resonance driving.

36. The method of operating the surgical robot apparatus according to claim 31, wherein the moving hand actuator comprises at least one magnetic driving gear, the moving hand manipulator comprises at least one magnetic induction gear corresponding to the magnetic driving gear, the magnetic induction gear is spatially contactlessly controlled by the magnetic driving gear.

37. The method of operating the surgical robot apparatus according to claim 31, wherein the surgical robot apparatus further comprises a track, when the moving hand actuator travels along the track, the moving hand manipulator is moved by spatially contactless control of the moving hand actuator.

38. The method of operating the surgical robot apparatus according to claim 37, wherein the moving hand actuator and the moving hand manipulator are each provided with magnetic components, magnetic attraction of the magnetic components is configured to engage the moving hand manipulator with the moving hand actuator together.

39. The method of operating the surgical robot apparatus according to claim 31, wherein the surgical robot apparatus further comprises a robot stationary hand, the robot stationary hand comprises a stationary hand holder on the sterile side and a controller on the non-sterile side, the stationary hand holder is spatially contactlessly controlled by the controller to hold or release the endo-tool.

40. The method of operating the surgical robot apparatus according to claim 39, wherein the operation method comprises: effectuating the robot stationary hand to be stationary between the robot moving hand and the clinical subject when the robot stationary hand holds the endo-tool stationary and the moving hand manipulator releases the endo-tool, the robot stationary hand moves towards or away from a clinical subject.
